# EUROPEAN PATENT SPECIFICATION

(11) **EP 4 247 448 B1**
(45) Date of publication and mention of the grant of the patent: **29.04.2026**
(21) Application number: 21806732.0
(22) Date of filing: 09.11.2021
(51) Int. Cl.: A61M 1/06

(54) **BREAST SHIELD FOR A BREAST PUMP**
BRUSTSCHUTZ FÜR EINE BRUSTPUMPE
CAPUCHON POUR SEIN POUR ENSEMBLE TIRE-LAIT

(30) Priority: 17.11.2020 EP 20208087
(43) Date of publication of application: 27.09.2023
(73) Proprietor: Koninklijke Philips N.V., 5656 AG Eindhoven (NL)
(72) Inventor: LIPSCH, Job, 5656 AE Eindhoven (NL); RABOTTI, Chiara, 5656 AE Eindhoven (NL); DOBRUSSKIN, Christoph, 5656 AE Eindhoven (NL); VAN DER KOOI, Johannes Tseard, 5656 AE Eindhoven (NL)
(74) Representative: Philips Intellectual Property & Standards
(86) International application number: PCT/EP2021/081024
(87) International publication number: WO 2022/106252

(56) References cited:
- EP-A1- 2 687 246
- EP-A1- 3 539 583
- US-A1- 2017 065 753
- US-A1- 2019 151 521
- US-A1- 2019 240 386

## Description

### FIELD OF THE INVENTION

The present invention relates to a breast shield for a breast pump to receive the breast of a user. The invention also relates to a breast pump comprising the shield of the invention.

### BACKGROUND OF THE INVENTION

Breast pumps are well known devices for extracting milk from a breast of a user. A breast pump may be used if the baby or infant is not itself able to extract milk from the breast, or if the mother is separated from the baby or infant and is to be fed with breast milk by someone else.

Breast pumps allow the nursing mother to express milk and provide collection of the breastmilk for later use. For some mothers, breast pumps may be a necessity, as when a child has suckling problems, or if the mother has problems with excessive or deficient milk production, or soreness, deformation or injury of the mammilla, or like conditions that are not conducive to suckling at the breast.

Breast pumps typically comprise a rigid, funnel-shaped shield connected to a vacuum pump having a container for collecting the milk. The funnel shaped shield is known as the breast shield. It is the interface between the user's breast and nipple with the pump and so its design is critical to maintaining the user's comfort whilst using the device. It is also important to ensure that the vacuum seal between the breast and the shield is maintained for optimal pumping.

The breast pump typically has two operating modes, namely a stimulation mode and an extraction mode. During the stimulation mode, the milk ejection reflex is stimulated. When the breast pump is activated, a pressure source such as a vacuum pump inside the breast pump generates an under-pressure and the stimulation mode can be started. The vacuum pump is typically driven by an electric motor. For portable breast pumps, these motors are driven by battery power.

Typical pressure profiles oscillate between a maximum negative pressure and then return to atmospheric pressure at the end of each cycle. By generating a pressure cycle, particularly a cycle using under-pressure levels below atmospheric pressure, a simulation of a feeding action is obtained, which triggers the necessary let-down reflex for lactating. However, a return to ambient pressure within the breast shield chamber may not be required, and benefits may be achieved by maintaining a certain level of under-pressure, i.e. a baseline vacuum, on the breast throughout at least a portion of the pumping session.

During placement of the breast shield, the mother has to correctly place the expression kit while at the same time having to turn on the vacuum pump (with the other hand). The nipple should be aligned as well as possible in the center of the breast shield. If the mother then turns on the vacuum pump, the breast shield often moves out of place. This problem becomes even more apparent if two expression kits are used at the same time, one for each breast.

While expressing milk, the breast shield or shields also need to be held in place during the entire duration of the session. Some dedicated bras are commercially available for hand-free use of the breast pumps. Besides increasing the cost of the expression set, an expression bra might not be as comfortable as a normal bra when needed to be worn for the whole day and women might need to undress before expressing milk.

Incorrect placement results in a number of disadvantages such as leakage and pain and trauma. If the breast shield is not correctly sealed against the breast, leakage can occur which can result in milk spilling over clothes or breastmilk waste. Pain and trauma may be caused by bruising to the nipple and areola.

To address this issue, breast pumps may also include an attachment mode before the breast pumping session, during which a low under-pressure level (i.e. a pressure close to atmospheric pressure) is applied to assist the user in positioning the breast shield. The use of an attachment mode is for example described in EP 3 151 876.

The pumping profile, baseline pressure and attachment pressure are usually controlled within a breast shield by acting on the same breast area (nipple and areola). This hampers comfort and the possibility of independently optimizing the different functions. US 2019/0151521 discloses a breast pump with two actuated bladders, one for stimulating a milk ejection reflex and one for extracting breast milk.

There is therefore a need for an improved design of breast shield to improve the ease of alignment.

### SUMMARY OF THE INVENTION

The invention is defined by the claims.

According to examples in accordance with an aspect of the invention, there is provided a breast shield for a breast pump, comprising:
a first cavity for placing over a first portion of the breast surface including the nipple of a breast, the first cavity coupled to a first pressure control port for controlling milk expression; and
a second cavity positioned radially outwardly from the first cavity, the second cavity coupled to a second pressure control port for providing a sealing, or positioning function, wherein the second cavity is for applying a pressure to a second portion of the breast surface radially outward from the first portion.

The first cavity is intended to cover the nipple, whereas the second cavity covers an area of the breast surface further radially outwardly spaced from the nipple. It is positioned radially outwardly, with reference to radial direction of the first cavity. This radial direction is perpendicular to the projection direction of the nipple, and perpendicular to a corresponding length axis of the first cavity.

The second cavity may have a closed shape (such as a circle or ellipse) surrounding the first cavity or it may have a shape which does not fully surround the first cavity. The use of separate pressure control ports means that different under-pressure levels and/or profiles may be applied to the different areas of the breast. For example, the pressure applied to the second portion of the breast may be a baseline under-pressure aimed at providing improved and easy placement whereas the pressure applied to the first portion is for the most efficient milk expression.

The breast shield thus has two separate compartments to enable this use of independent pressure profiles. The second cavity may be formed outside the normal size of a breast shield. **In** this case, the breast shield provides a larger surface of contact. Alternatively, the first cavity may have a reduced sized compared to a conventional breast shield so that the overall size remains approximately the same.

When the second has a closed shape around the first cavity, the breast shield has one circular (or oval) first cavity for providing an expression pressure profile and a concentric second cavity in the shape of a corresponding closed shape.

The second cavity may comprise an open shape which may be only partially around the first cavity. Thus, a full closed shape is not needed. The size of the second cavity only needs to be sufficient to perform the intended function, which is for example a holding function. It may be simply to one side of the first cavity, e.g. above the first cavity.

The first cavity may have a milk output channel and the second cavity is a shape with an interruption located at the milk output channel. Thus, the second cavity does not complicate the connections to the first cavity.

The second cavity may have a covering membrane. This is used to protect the vacuum pump system from dirt and debris and makes the breast shield easier to clean. The membrane forms an interface between the second cavity and the breast. This may also give more comfort to the user.

There may be one or more further cavities, so that there is a set of cavities radially outside the first cavity, for example a set of two or more concentric outer cavities. A pressure may be switched between the multiple chambers defined by these outer cavities to maintain a constantly applied under-pressure to hold the breast pump while not having a constant under-pressure on any particular area of the breast tissue. This avoids bruises that can occur when an under-pressure is constantly applied to a same position. **In** this way, the breast shield further comprises at least one further cavity positioned radially outwardly of the second cavity.

The second cavity may be divided into segments, wherein each segment has its own pressure control port. Some of these pressure control ports may be coupled together so that the segments are grouped, or they may be independent.

The use of multiple segments, to which independent and adaptive pressure profiles may be applied, gives the option of massaging the breast during milk expression. Massaging the breast during milk expression has been demonstrated to increase the quality of breast milk. The combination of suckling and massaging also assists in emptying the milk ducts as well encouraging the production of more milk.

The second cavity maybe rigidly mounted to the first cavity. They may for example be formed as a single molded unit.

Alternatively, the second cavity may be elastically non-rigidly mounted to the first cavity. There may for example be an elastic ring between them. This improves user comfort by enabling the breast shield to adapt at least partly to the shape of the breast.

A ridge between the inner and outer cavity is preferably designed to maximize comfort, by avoiding high friction surfaces, hard materials or sharp edges. This ridge functions as the main seal for preventing leakage of milk from the first cavity into the second cavity. The second cavity is not intended to function as a milk collection chamber.

The second cavity may be non-circular with a greater height than width. The height is the axis which is intended to be aligned with the height axis of the user, i.e. intended to be vertical in normal use. It may for example be oval. This means that more of the suction force is acting against the momentum resulting from the weight of the wearable part multiplied by the distance to the center of gravity, so providing a more effective holding of the breast shield. This results in improved fitting.

The invention also provides a breast pump comprising:
a vacuum pump system;
an expression kit which comprises the breast shield as defined above coupled to the vacuum pump system; and
a controller for controlling the vacuum pump system.

The vacuum pump system may comprise a vacuum pump, and a pressure regulator system for delivering a pressure to the first cavity and the second cavity. Thus, a single vacuum pump may be used, and the output is regulated by a regulator system (e.g. separate pressure regulators) to create at least two different pressure profiles.

The vacuum pump system may instead comprise a first vacuum pump for the first cavity (for the expression function) and a second vacuum pump for the second cavity (e.g. for creating a baseline pressure).

The controller may be adapted to apply a holding vacuum to the second cavity which adapts over time from an intermittent application of under-pressure to a constant application of under-pressure when the breast shield is first positioned.

Alternatively, a constant but low initial holding under-pressure may be used when the breast shield is first positioned.

The intermittent application of under-pressure and/or the use of a low under-pressure enables a user to reposition the breast shield initially, and the breast shield is then held more constantly when the constant or higher under-pressure is applied.

The controller may be adapted to apply a massage pressure profile to the second cavity during milk expression using the first cavity. This may for example make use of a segmented second cavity as discussed above.

The breast pump system may comprise a user interface to allow a user to select pressure characteristics for the first and second cavities. Thus, the user may prefer to have the attachment performed by the expression pressure (as in a conventional breast pump) and may thus choose to disable the pressure applied to the second cavity. Thus, the system can be configured to operate in different ways, including operating in the same way as conventional breast pumps.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a first example of a breast shield;
Figure 3 shows a first implementation of the example of Figure 2;
Figure 4 shows a second implementation of the example of Figure 2;
Figure 5 shows a second example of a breast shield;
Figure 6 shows a third example of a breast shield;
Figure 7 shows a fourth example of a breast shield;
Figure 8 shows two designs with additional outer cavities;
Figure 9 shows a fifth example of a breast shield;
Figure 10 shows an example of an intermittent application of under-pressure; and
Figure 11 shows a design for the seal provided between the first and second cavities.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast shield for a breast pump, which comprises a first cavity for placing over the nipple and having a first pressure control port for controlling milk expression. A second cavity is radially outwardly positioned relative to first cavity (e.g. around or partially around the first cavity) and is coupled to a second pressure control port for providing a sealing or positioning function. Thus, a positioning/holding function and a milk expression function may be optimized independently.

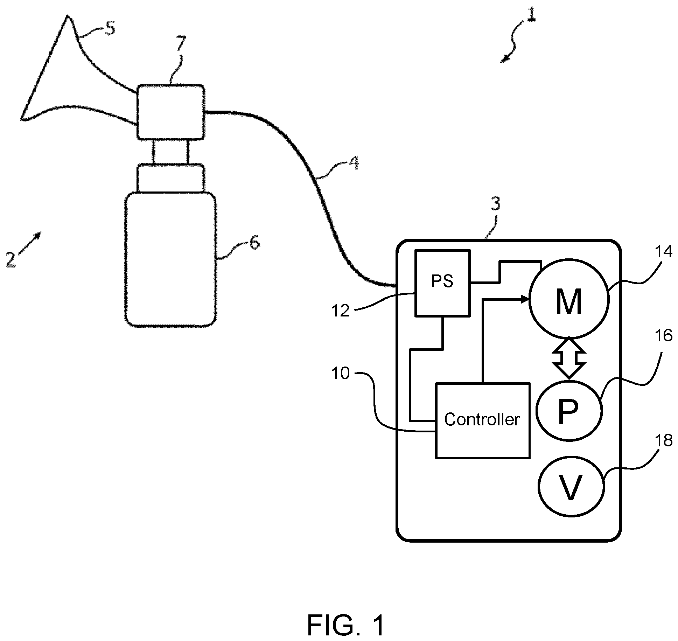

Figure 1 shows a known breast pump system 1, comprising an expression unit 2 and a pump arrangement 3 which are connected via a tube 4. The pump arrangement includes various components in addition to the pump, so may be considered to be a general operating unit.

The expression unit 2 is formed with a main body 7, a funnel 5 for receiving a breast of a user and a receptacle 6 for collecting the expressed milk. The funnel 5 is commonly known as a breast shield. The breast shield 5 and the receptacle 6 are connected to the main body 7. The main body 7 comprises a vacuum chamber, i.e. a chamber to which any desired under-pressure may be applied. A flexible membrane or diaphragm is located in the vacuum chamber, and this membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The controller controls 10 the operation of the power source 12, motor 14 and vacuum pump 16. The vacuum pump delivers an under-pressure at one side and an over-pressure at an opposite side (relative to a middle reference pressure, which may be the atmospheric pressure). The pump arrangement 3 further comprises a solenoid valve 18.

In use, the vacuum pump applies an under-pressure to the membrane located in the main body 7 so that it deforms. The membrane deforms to create an under-pressure in the breast shield 5 which in turns applies an under-pressure to the breast which enables milk to be expressed.

Although the breast pump system 1 is described as comprising a membrane such that the under-pressure is applied indirectly to the breast, it should be understood that in an alternative embodiment, the under-pressure is applied directly to the breast of a user. In this case, the breast pump system does not comprise a membrane and the under-pressure created by the vacuum pump is applied directly to the breast.

The under-pressure is applied to the breast at intervals. That is, a pressure differential is applied on a cyclic basis. After an under-pressure has been established, the pressure from the vacuum pump is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates an under-pressure in the expression unit which enables milk to be expressed from the breast of a user. When the solenoid valve is opened, the under-pressure generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

The invention relates in particular to the breast shield 5. The breast shield needs to be correctly aligned on the breast and it also needs to remain in place during milk expression. **In** conventional systems, these functions all have to be achieved by the pressure profile applied to the single area of the breast within the breast shield.

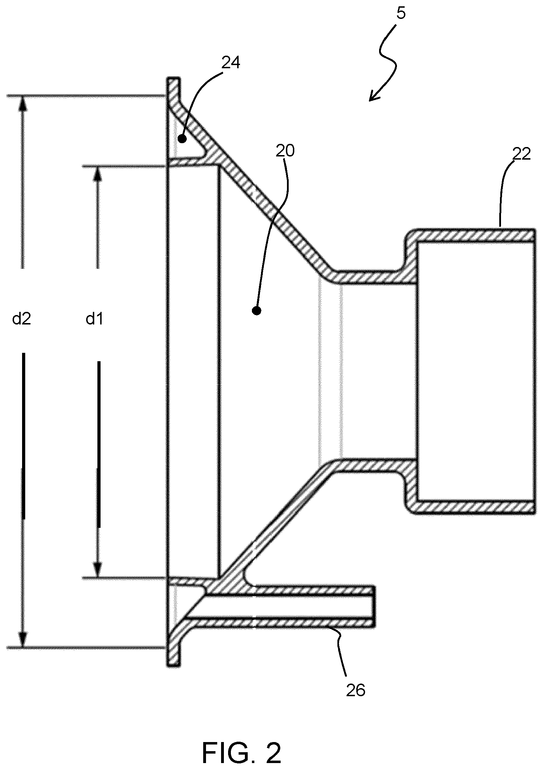

Figure 2 shows a first example of a breast shield 5 in accordance with the invention. The breast shield 5 has a first cavity 20 for placing over the nipple. This will be termed a "first portion" of the breast surface. It has a closed shape such as circle around the nipple. It will be described as an inner cavity in the following description. The inner cavity 20 is coupled to a first pressure control port 22 for applying a first pressure profile to the first cavity, in particular for controlling the milk expression. This first pressure profile is thus applied to the nipple.

A second cavity 24 extends at least partially around the outside of the inner cavity 20. It will be described as an outer cavity in the following description. The outer cavity is coupled to a second pressure control port 26 for applying a second pressure profile to the outer cavity, in particular for providing a sealing, or positioning function. The outer cavity interfaces with a second portion of the breast surface which extends at least partially around the first portion. This second portion is typically a ring of breast tissue outside the area of the areole.

The first pressure profile may be considered to implement the basic functionality of the breast pump and the second pressure profile may be considered to implement supporting functions. The two functions may be operated independently, in space and time. Inside the breast shield, the basic functionality focuses on efficient milk extraction e.g. the pumping profile and baseline under-pressure. The supporting functions are for example ensuring a good seal to provide leakage prevention, and to provide attachment of the breast shield and optionally a separate hold and assist function during initial placement of the breast shield.

**In** one example, the inner cavity has an inner diameter d1 such as to fit an average range of nipple/teat sizes while the outer diameter d2 is similar to the existing diameters of the breast shield.

The inner diameter d1 for example may range from 20mm to 35mm and the outer diameter d2 may range from 10cm to 15cm.

The surface area between d1 and d2 is proportional to the force exerted by the cup due to the applied under-pressure.

Alternatively, the inner diameter may be the same as the existing diameter of a breast shield, and the second cavity results in an increased size.

The diameter ratio and pressure ratio between the cavities can differ in relation to the amount of desired under-pressure.

While the physical separation into two compartments mainly concerns the basic and support functions for the under-pressure, atmospheric pressure and over pressure, a separation in time enables additional user interface and user experience (UI/UX) functionality.

For example, the independent control of the two pressure profiles enables personalization and adjustability based on user preferences but also allows adjusting the pressure profiles independently of each other. For example, there may be the option to have direct attachment using the second cavity without applying a baseline vacuum to the nipple and areola. Alternatively, the holding function may, as in conventional breast pump, be implemented by the inner cavity.

The attachment force for holding the breast shield on the breast is achieved using a minimum baseline under-pressure in the second cavity, intended to mimic the baseline vacuum naturally created by the baby's mouth between the tongue and palate for attachment.

The contact area and level of negative pressure determine the force required to balance the weight of the breast shield and any connected components, and hold it in place. Ideally, the second cavity is able to support the full weight of the device alone and/or in combination with the first, internal cavity. This may be achieved with different vacuum levels in the two compartments.

As mentioned above, the user may choose not to use the option of direct attachment using the second cavity.

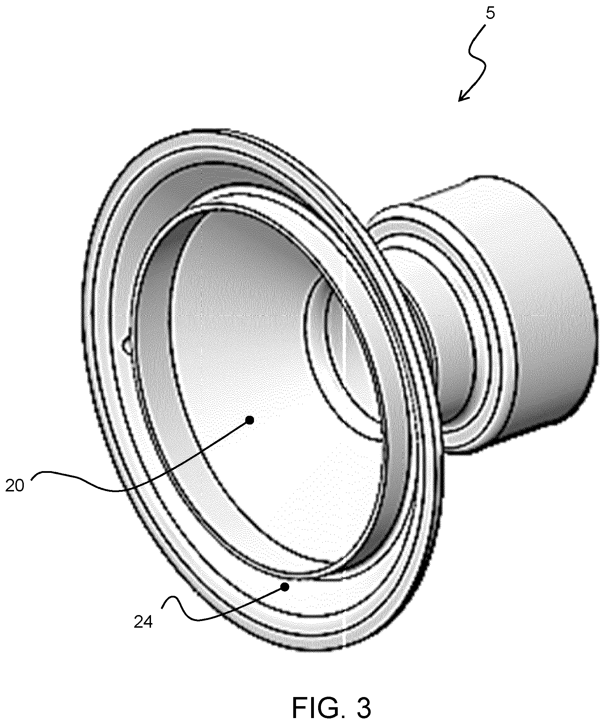

Figure 3 shows a first implementation of the example of Figure 2 in which the outer cavity 24 comprises a closed shape around the inner cavity.

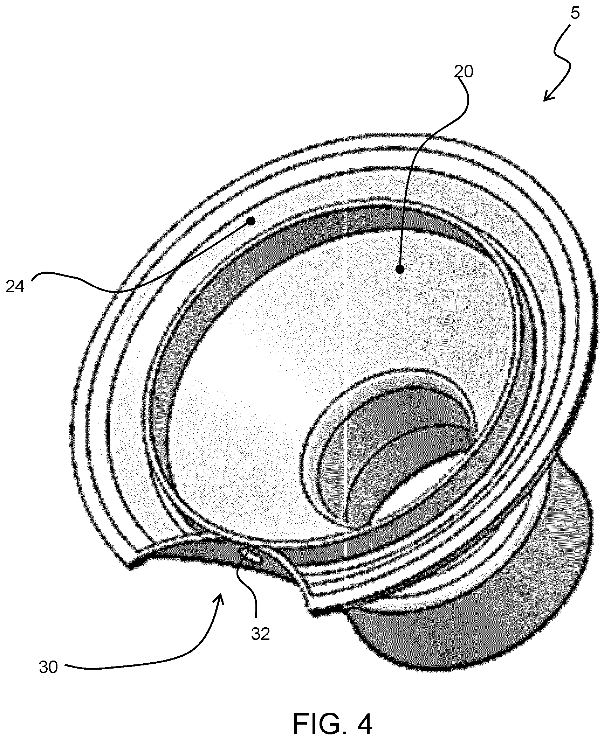

Figure 4 shows a second implementation of the example of Figure 2 in which the outer cavity 24 comprises a shape only partially around the inner cavity.

The second cavity is thus open, having a C-shape. This enables connections to the first cavity bypassing the direct attachment of the outer compartment. For example, a milk transport passage may extend from the first cavity towards the bottle through the interruption 30. The inner cavity 22 then has a milk output channel 32 and the outer cavity has an interruption 30 located at the milk output channel.

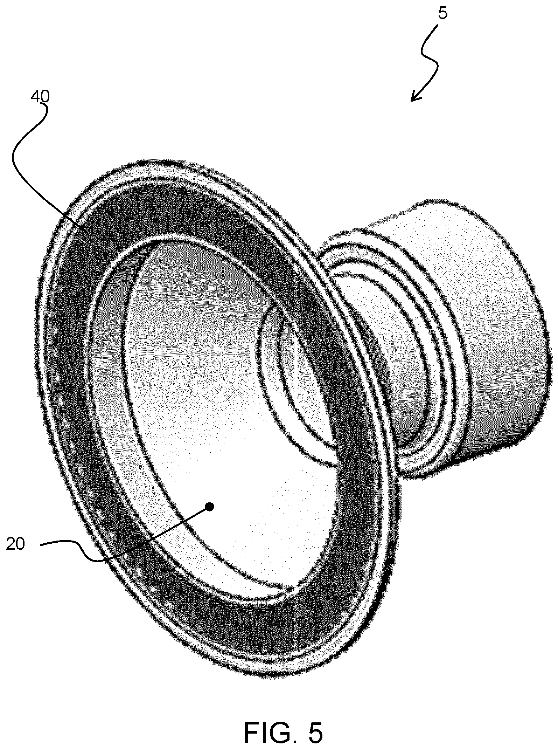

The outer cavity may be smaller and hence located at one location around the outer periphery of the inner cavity, e.g. at the top
Figure 5 shows an example in which the outer cavity has a covering membrane 40.

The example of Figures 2 to 4 are open systems, meaning the under-pressure or other pressure is in direct contact with the skin. This might be undesirable when there is a rapid application of under-pressure or if there is a risk of milk being sucked into the vacuum system. The membrane 40 functions as an active suction cup to create an attachment on the breast.

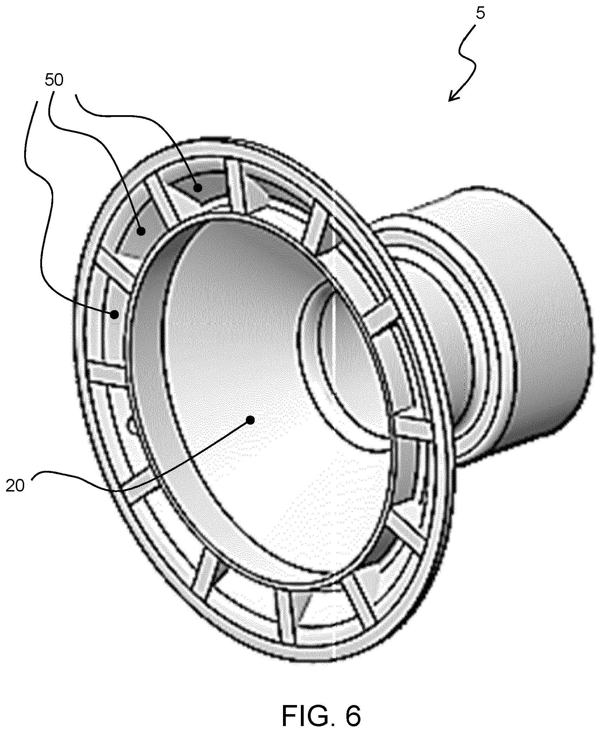

Figure 6 shows an example in which the outer cavity is separated into segments 50, wherein each segment has its own pressure control port (not shown). The segments may be controlled individually or in groups. Thus, each segment has a pressure control port so that a pressure may be applied to the chamber formed by the segment.

A single covering membrane as shown in Figure 5 may cover the set of separated segments of the design of Figure 6.

Having multiple segments for example enables the use of over pressure in one or more segments while the other segments secure the breast shield by an applied under-pressure. By having a balance between the under-pressure of some segments and the over pressure of other segments, a number of over pressure profiles can provide massaging, by circular motion or within a specific target area, to improve the milk extraction while the remaining segments still hold the breast shield secure to the breast.

A massage function for example requires a difference in pressure between different points, which varies over time. The massage function may use an over-pressure or an under-pressure or a combination.

By changing the actuation of the segments, the over pressure and massage function can move around the outer diameter of the breast. There may again be a membrane between the applied pressure and the skin.

The examples above all form the two cavities using a single integrated molded component so that the outer cavity is rigidly mounted to the inner cavity.

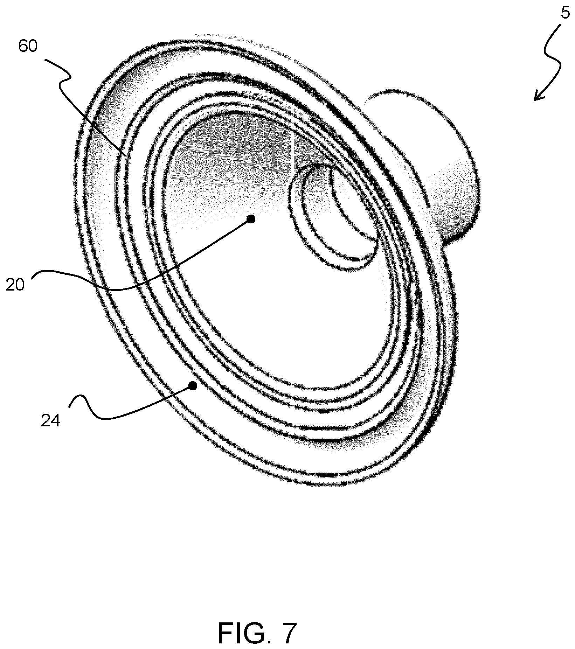

Figure 7 shows an example in which the outer cavity 24 is elastically non-rigidly mounted to the inner cavity 22 by means of an elastic ring 60. This elastic ring may form part of the tubing of the breast shield. This reduces the attachment to the remainder of the breast shield and provides a suspension. This makes the outer cavity at least partially mechanically independent from the central body of the breast shield, so that movement can be supported between the two cavities to allow a best fit to the breast.

The examples above have a single inner cavity and a single outer cavity. There may instead be multiple outer cavities.

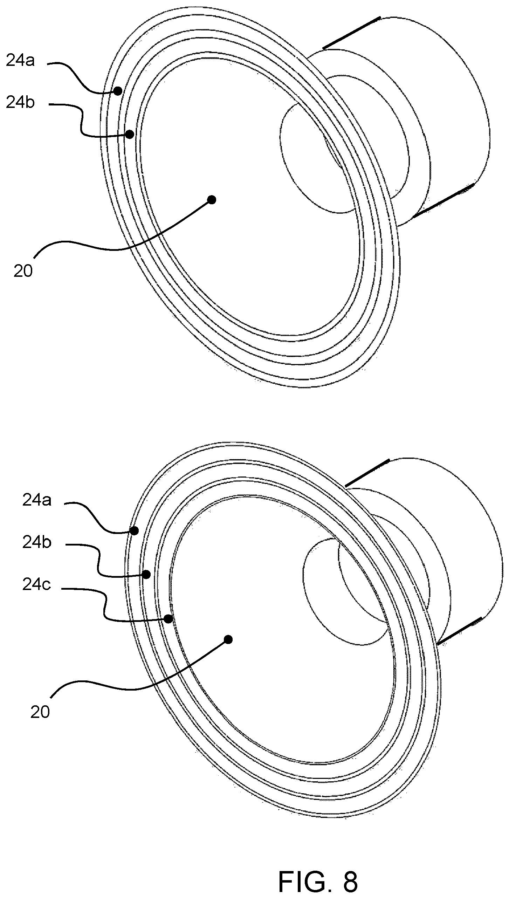

Figure 8 shows in the top image two concentric outer cavities 24a, 24b around the inner cavity. Figure 8 shows in the bottom image three concentric outer cavities 24a, 24b, 24c around the inner cavity.

By switching the application of under-pressure between the multiple outer cavities, it can be ensured that there is a constantly present under-pressure to hold the breast pump while there is no constant under-pressure all of the tissue. This avoid bruises that can occur when an under-pressure is constantly applied to one location of the skin. The pressures could for example follow a sinusoidal pattern, with opposite phase between the two outer cavities 24a,24b, or a three phase approach for three outer cavities.

The positioning of the breast shield needs to overcome the force of gravity.

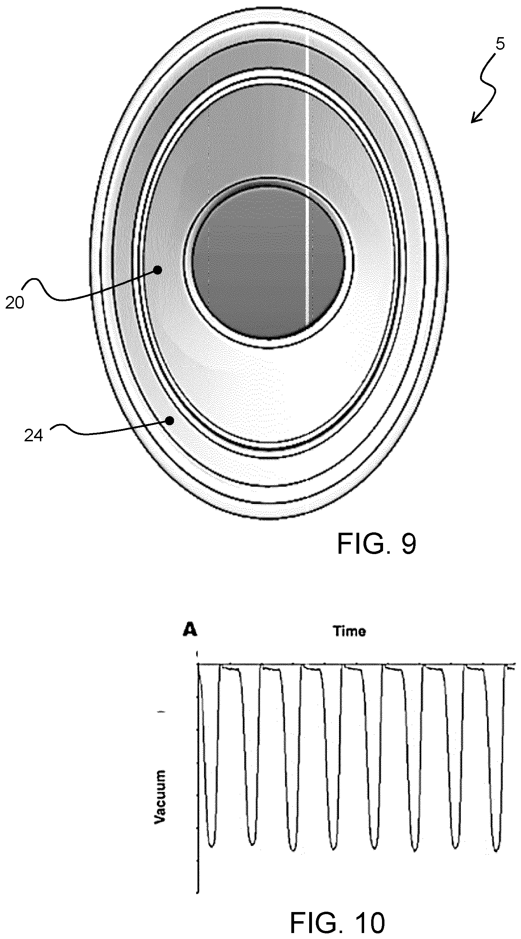

Figure 9 shows an example in which at least the outer cavity is non-circular with a greater height than width. This means that the force component exerted by the second cavity in the vertical direction (parallel to gravity) is stronger than the horizontal component. An oval or rectangular shape may be used. This gives the advantage that in the vertical direction more force is available, avoiding unintended movement, while horizontal movements, which are less influenced by gravity, are facilitated for improved comfort and fit.

The sum of the forces resulting from the under-pressure levels applied to the first and second cavities preferably remains at least at a level sufficient to hold the expression kit to the breast, thereby counteracting gravity.

The oval design may be combined with any of the previous examples. When the inner and outer cavities are concentric, they will both have the same non-circular shape.

**In** order to provide different under-pressure levels and/or profiles to the two cavities (or indeed the multiple segments for a segmented second cavity), a single vacuum pump may be used but with a regulator system which controls the pressure applied to first cavity and the second cavity. A buffer and valve system may be used to regulate the pressures in the cavities from a share vacuum pump. For example, EP 2 598 183 discloses a vacuum pump and a variable volume buffer volume, in which the buffer volume may be used to control a negative pressure generated. This type of system may be used for each of the cavities.

Alternatively, there may be a first vacuum pump for the first cavity and a second vacuum pump for second cavity.

As explained above, the second cavity may have the function of providing a holding force. A holding vacuum may be applied to the outer cavity which adapts over time from an intermittent application of under-pressure to a constant application of under-pressure when the breast shield is first positioned.

Placing a breast shield with a fixed pre-established constant vacuum results in a quick positioning of the device on the breast. However, a constant baseline vacuum is not optimal for repositioning, as the user needs to break the vacuum seal before repositioning into the desired position. The use of an intermittent short holding (vacuum) and a release pressure (a lower vacuum, or atmospheric pressure or even an over pressure) means the user can reposition gradually until she feels that the positon is optimal. The rhythm or pace of the intermitting pressure and release changes to a full vacuum suitable for holding and operating the device hands-free.

Figure 10 shows the intermittent application of under-pressure as pressure (relative to atmospheric pressure) versus time. The intermittent pressure enables repositioning by the user until the correct alignment is found.

The switch to a constant vacuum may be after a fixed time, or user specified time, or when an input command is received from the user, the pressure can convert to a fixed baseline pressure.

The controller may also apply a massage pressure profile to the outer cavity during milk expression using the first cavity.

A user interface may allow a user to select pressure characteristics for the first and second cavities as mentioned above. This provides personalization and adjustment to user preferences. Direct attachment using the second cavity means that the pumping vacuum profile does not need the conventional baseline vacuum on the nipple and areola.

In the examples above, there is an annular ridge between the cavities, and this ridge is held against the skin by the under-pressure in one or both of the cavities on each side. This ridge may be adapted to ensure the best possible comfort for the wearer. For example, instead of a flat surface with a sharp edges leading into the cavities on each side, a curved shape may be formed, with a soft low friction surface where there will be contact with the skin. By providing a curved shape for the wall between the cavities, sharp edges can be avoided.

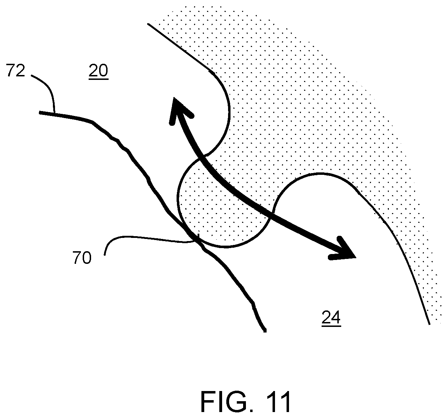

Figure 11 shows an example of a design of the interface between the inner and outer cavities. The ridge 70 has a smooth curved surface. In addition, it can deflect as represented by the arrow so that it can move in response to movement of the skin surface 72 for example induced by the different pressures applied to the cavities. In this way, the ridge can remain in contact with the same area of skin and does not need to slide relative to the skin surface when there are small movements in the skin surface. The ridge can move with the skin.

A curved shape may be formed by an integrated molded shape feature, but it may instead be an externally applied pad which is mounted over the front face of the breast shield.

The curved shape between the cavities may be formed by a flexible wall portion which defines the transition between adjacent cavities. It may then define a soft sealing ring which separates a first cavity from a second cavity.

The invention has been described above with reference to a conventional vacuum pump (motor and impeller) and solenoid. However, there are other options for creating the desired under-pressure, such as hydraulic-driven systems, ultrasonic motors, and peristaltic pumps. Any of these different ways of producing an under-pressure may be used for the inner and/or outer cavities.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast shield (5) for a breast pump, comprising:
a first cavity (20) for placing over a first portion of the breast surface including the nipple of a breast, the first cavity having a maximum diameter (d1) where the breast shield is to contact the breast surface, the maximum diameter (d1) thereby defining the extent of the first portion of the breast surface, the first cavity coupled to a first pressure control port (22) for controlling milk expression; and
a second cavity (24) positioned radially outwardly from the first cavity, the second cavity coupled to a second pressure control port (26) for providing a sealing, or positioning function, wherein the second cavity is for applying a pressure to a second portion of the breast surface radially outward from the first portion.

2. The breast shield of claim 1, wherein the second cavity extends at least partially around the outside of the first cavity.

3. The breast shield of claim 1, wherein the second cavity (24) comprises:
a closed shape around the first cavity; or
an open shape extending partially around the first cavity.

4. The breast shield of claim 3, wherein the first cavity (20) has a milk output channel (32) and the ring has an interruption (30) located at the milk output channel.

5. The breast shield of any one of claims 1 to 4, wherein the second cavity (24) has a covering membrane (40).

6. The breast shield of any one of claims 1 to 5, wherein the second cavity (24) is separated into segments, wherein each segment has its own pressure control port.

7. The breast shield of any one of claims 1 to 6, wherein:
the second cavity (24) is rigidly mounted to the first cavity (20); or
the second cavity (24) is elastically non-rigidly mounted to the first cavity (20).

8. The breast shield of any one of claims 1 to 7, further comprising at least one further cavity (24a, 24b) positioned radially outwardly of the second cavity (24c).

9. The breast shield of any one of claims 1 to 8, wherein the second cavity (24) is non-circular with a greater height than width.

10. A breast pump comprising:
a vacuum pump system (14,16);
an expression kit (2) which comprises the breast shield of any one of claims 1 to 9 coupled to the vacuum pump system (14,16); and
a controller (10) for controlling the vacuum pump system.

11. The breast pump system of claim 10, wherein the vacuum pump system comprises a vacuum pump (16), and a regulator system for controlling the pressure applied from the vacuum pump to the first and second cavities.

12. The breast pump system of claim 10, wherein the vacuum pump system comprises a first vacuum pump for the first cavity and a second vacuum pump for the second cavity.

13. The breast pump system of any one of claims 10 to 12, wherein the controller (10) is adapted to apply a holding under-pressure to the second cavity which adapts over time from an intermittent application of a particular under-pressure level to a constant application of under-pressure when the breast shield is first positioned.

14. The breast pump system of any one of claims 10 to 13, wherein the controller (10) is adapted to apply a massage pressure profile to the second cavity during milk expression using the first cavity.

15. The breast pump system of any one of claims 10 to 14, comprising a user interface to allow a user to select pressure characteristics for the first and second cavities.

## Patentansprüche

1. Brustschutz (5) für eine Milchpumpe, umfassend:
eine erste Vertiefung (20) zur Platzierung über einem ersten Abschnitt der Brustoberfläche einschließlich der Brustwarze einer Brust, wobei die erste Vertiefung einen maximalen Durchmesser (dl) an der Stelle aufweist, an der der Brustschutz die Brustoberfläche berührt, wobei der maximale Durchmesser (dl) dadurch die Ausdehnung des ersten Abschnitts der Brustoberfläche definiert, wobei die erste Vertiefung mit einem ersten Drucksteueranschluss (22) zur Steuerung des Milchabpumpens verbunden ist; und
eine zweite Vertiefung (24), die radial außerhalb der ersten Vertiefung positioniert ist, wobei die zweite Vertiefung (24) mit einem zweiten Drucksteueranschluss (26) verbunden ist, um eine Dichtungs- oder Positionierungsfunktion bereitzustellen, wobei die zweite Vertiefung dazu dient, Druck auf einen zweiten Abschnitt der Brustoberfläche auszuüben, der radial nach außen vom ersten Abschnitt liegt.

2. Brustschutz nach Anspruch 1, wobei sich die zweite Vertiefung zumindest teilweise um die Außenseite der ersten Vertiefung herum erstreckt.

3. Brustschutz nach Anspruch 1, wobei die zweite Vertiefung (24) Folgendes umfasst:
eine geschlossene Form um die erste Vertiefung herum; oder
eine offene Form, die sich teilweise um die erste Vertiefung herum erstreckt.

4. Brustschutz nach Anspruch 3, wobei die erste Vertiefung (20) einen Milchauslasskanal (32) aufweist und der Ring eine Unterbrechung (30) aufweist, die sich im Milchauslasskanal befindet.

5. Brustschutz nach einem der Ansprüche 1 bis 4, wobei die zweite Vertiefung (24) eine Deckmembran (40) aufweist.

6. Brustschutz nach einem der Ansprüche 1 bis 5, wobei die zweite Vertiefung (24) in Segmente unterteilt ist, wobei jedes Segment seinen eigenen Drucksteueranschluss aufweist.

7. Brustschutz nach einem der Ansprüche 1 bis 6, wobei:
die zweite Vertiefung (24) starr an der ersten Vertiefung (20) angebracht ist; oder
die zweite Vertiefung (24) elastisch und nicht starr an der ersten Vertiefung (20) angebracht ist.

8. Brustschutz nach einem der Ansprüche 1 bis 7, weiter umfassend zumindest eine weitere Vertiefung (24a, 24b), die radial außerhalb der zweiten Vertiefung (24c) positioniert ist.

9. Brustschutz nach einem der Ansprüche 1 bis 8, wobei die zweite Vertiefung (24) nicht kreisförmig ist und eine größere Höhe als Breite aufweist.

10. Milchpumpe, umfassend:
ein Vakuumpumpensystem (14, 16);
ein Abpumpset (2), das den Brustschutz nach einem der Ansprüche 1 bis 9 umfasst, der mit dem Vakuumpumpensystem (14,16) verbunden ist; und
eine Steuereinheit (10) zum Steuern des Vakuumpumpensystems.

11. Milchpumpensystem nach Anspruch 10, wobei das Vakuumpumpensystem eine Vakuumpumpe (16) und ein Regelsystem zur Steuerung des von der Vakuumpumpe auf die erste und zweite Vertiefung ausgeübten Drucks umfasst.

12. Milchpumpensystem nach Anspruch 10, wobei das Vakuumpumpensystem eine erste Vakuumpumpe für die erste Vertiefung und eine zweite Vakuumpumpe für die zweite Vertiefung umfasst.

13. Milchpumpensystem nach einem der Ansprüche 10 bis 12, wobei die Steuereinheit (10) dazu angepasst ist, um einen Halteunterdruck auf die zweite Vertiefung auszuüben, der sich im Laufe der Zeit von einer intermittierenden Anwendung eines bestimmten Unterdruckniveaus zu einer konstanten Anwendung eines Unterdrucks anpasst, wenn der Brustschutz zum ersten Mal positioniert wird.

14. Milchpumpensystem nach einem der Ansprüche 10 bis 13, wobei die Steuereinheit (10) dazu angepasst ist, während des Abpumpens von Milch unter Verwendung der ersten Vertiefung ein Massagedruckprofil auf die zweite Vertiefung auszuüben.

15. Milchpumpensystem nach einem der Ansprüche 10 bis 14, umfassend eine Benutzerschnittstelle, die es dem Benutzer ermöglicht, Druckcharakteristika für die erste und zweite Vertiefung auszuwählen.

## Revendications

1. Téterelle (5) pour un tire-lait, comprenant :
une première cavité (20) destinée à recouvrir une première partie de la surface du sein, incluant le mamelon, la première cavité présentant un diamètre maximal (dl) à l'endroit où la téterelle est en contact avec la surface du sein, le diamètre maximal (dl) définissant, de ce fait, l'étendue de la première partie de la surface du sein, la première cavité étant couplée à un premier orifice de régulation de pression (22) pour réguler l'expression du lait ; et
une seconde cavité (24) positionnée radialement vers l'extérieur de la première cavité, la seconde cavité étant couplée à un second port de régulation de pression (26) pour assurer une fonction d'étanchéité ou de positionnement, dans laquelle la seconde cavité est destinée à appliquer une pression à une seconde partie de la surface du sein radialement vers l'extérieur de la première partie.

2. Téterelle selon la revendication 1, dans laquelle la seconde cavité s'étend au moins partiellement autour de l'extérieur de la première cavité.

3. Téterelle selon la revendication 1, dans laquelle la seconde cavité (24) comprend :
une forme fermée autour de la première cavité ; ou
une forme ouverte s'étendant partiellement autour de la première cavité.

4. Téterelle selon la revendication 3, dans laquelle la première cavité (20) présente un canal de sortie de lait (32) et l'anneau présente une interruption (30) située au niveau du canal de sortie de lait.

5. Téterelle selon l'une quelconque des revendications 1 à 4, dans laquelle la seconde cavité (24) présente une membrane de recouvrement (40).

6. Téterelle selon l'une quelconque des revendications 1 à 5, dans laquelle la seconde cavité (24) est divisée en segments, dans laquelle chaque segment présente son propre orifice de régulation de pression.

7. Téterelle selon l'une quelconque des revendications 1 à 6, dans laquelle :
la seconde cavité (24) est montée de manière rigide sur la première cavité (20) ; ou
la seconde cavité (24) est montée de manière élastique non rigide sur la première cavité (20).

8. Téterelle selon l'une quelconque des revendications 1 à 7, comprenant en outre au moins une cavité supplémentaire (24a, 24b) positionnée radialement à l'extérieur de la seconde cavité (24c).

9. Téterelle selon l'une quelconque des revendications 1 à 8, dans laquelle la seconde cavité (24) est non circulaire avec une hauteur plus grande que la largeur.

10. Tire-lait comprenant :
un système de pompe à vide (14, 16) ;
un kit d'expression (2) comprenant la téterelle selon l'une quelconque des revendications 1 à 9, couplé au système de pompe à vide (14, 16) ; et
un dispositif de commande (10) pour commander le système de pompe à vide.

11. Système de tire-lait selon la revendication 10, dans lequel le système de pompe à vide comprend une pompe à vide (16) et un système de régulation pour réguler la pression appliquée par la pompe à vide aux première et seconde cavités.

12. Système de tire-lait selon la revendication 10, dans lequel le système de pompe à vide comprend une première pompe à vide pour la première cavité et une seconde pompe à vide pour la seconde cavité.

13. Système de tire-lait selon l'une quelconque des revendications 10 à 12, dans lequel le dispositif de commande (10) est adapté pour appliquer une sous-pression de maintien à la seconde cavité qui s'adapte au fil du temps d'une application intermittente d'un niveau de sous-pression particulier à une application constante de sous-pression lorsque la téterelle est positionnée pour la première fois.

14. Système de tire-lait selon l'une quelconque des revendications 10 à 13, dans lequel le dispositif de commande (10) est adapté pour appliquer un profil de pression de massage à la seconde cavité pendant une expression du lait à l'aide de la première cavité.

15. Système de tire-lait selon l'une quelconque des revendications 10 à 14, comprenant une interface utilisateur pour permettre à une utilisatrice de sélectionner des caractéristiques de pression pour les première et seconde cavités.
